# EUROPÄISCHE PATENTSCHRIFT

(11) **EP 0 933 130 B1**
(45) Veröffentlichungstag und Bekanntmachung des Hinweises auf die Patenterteilung: **06.10.2004**
(21) Anmeldenummer: 99100776.6
(22) Anmeldetag: 16.01.1999
(51) Int. Cl.: B01J 23/66, B01J 37/02, B01J 37/08, C07D 301/10

(54) **Verfahren zur Herstellung von silberhaltigen Katalysatoren und Katalysator-Zwischenprodukte und deren Verwendung für die Alkylenoxid-Herstellung**
Process for preparing supported silver-containing catalysts and catalyst precursors and their use in the alkylene oxide production
Procédé de préparation de catalyseurs supportés à base d'argent et leurs précurseurs et leur utilisation dans la production d'oxyde d'alkylène

(30) Priorität: 31.01.1998 DE 19803890
(43) Veröffentlichungstag der Anmeldung: 04.08.1999
(73) Patentinhaber: BP Köln GmbH, D-50769 Köln (DE)
(72) Erfinder: Grub, Joachim Dr., 41539 Dormagen (DE); Volprecht, Werner, 50129 Bergheim (DE); Baum, Matthias Dr., 41542 Dormagen (DE); Reimer, Alfred Dr., 41539 Dormagen (DE)
(74) Vertreter: Weber, Thomas, Dr.Dipl.-Chem.

(56) Entgegenhaltungen:
- EP-A- 0 428 845
- US-A- 4 305 844
- US-A- 4 400 308

## Beschreibung

Die Erfindung betrifft ein Verfahren zur Herstellung silberhaltiger und gegebenenfalls Promotoren enthaltender Trägerkatalysatoren und Katalysator-Zwischenprodukte und ihre Verwendung zur Herstellung von Alkylenoxiden durch direkte Oxidation von Alkenen mit Luft bzw. Sauerstoff enthaltenden Gasen.

Für die industrielle Herstellung von Alkylenoxiden verwendet man heute die Direktoxidation von Alkenen mit Luft bzw. mit molekularen Sauerstoff enthaltenden Gasen in Gegenwart eines silberhaltigen Katalysators. Da Alkylenoxide, besonders Ethylenoxid, als Basischemikalien für zahlreiche Folgeprodukte eine große wirtschaftliche Bedeutung haben, sind viele Versuche unternommen worden, die Leistungsfähigkeit der eingesetzten Katalysatoren ständig zu verbessern. Die zahlreichen vorgeschlagenen Modifikationen zur Verbesserung der Aktivität und der Selektivität betreffen das verwendete Trägermaterial, das Verfahren zur Herstellung der Katalysatoren und den Zusatz von Promotoren (R. Landau und R.E. Lidow, Ethylene and its industrial derivatives" Verlag S.A. Miller und Ernest Benn, London 1963; D.J. Hucknall Selektiv oxidations of hydrocarbons" Academic Press, London 1974; J. of catalysis 34, 100 bis 114 (1974)).

Besondere Bedeutung kommt der Arbeitstemperatur eines Katalysators für die Alkylenoxid-Herstellung zu. Wünschenswert sind Katalysatoren, die bei niedriger Temperatur eine hohe Aktivität und Selektivität aufweisen. Eine niedrige Arbeitstemperatur bewirkt beispielsweise eine längere Standzeit für den Katalysator, was für das technische Verfahren eine große Bedeutung hat. Weiterhin ist die Bildung von Nebenprodukten, beispielsweise bei der Ethylenoxid-Herstellung die Bildung des isomeren Acetaldehyds und des Formaldehyds, bei niedrigen Temperaturen wesentlich geringer und erleichtert somit die Abtrennung dieser Verunreinigungen bei der Aufarbeitung des Alkylenoxids zu einer reinen, allen Ansprüchen genügenden Ausgangschemikalie.

Bei hohen Arbeitstemperaturen kommt es neben den bereits geschilderten Nachteilen oftmals am Ausgang des Katalysatorbettes zu unerwünschten Nachreaktionen. Die dabei auftretenden Reaktionsprodukte können den Katalysator in seiner Leistungsfähigkeit schädigen und führen weiterhin zu unerwünschten Produktionsausfällen in kommerziell genutzten Anlagen. Hohe Arbeitstemperaturen begünstigen weiterhin das Auftreten von unkontrollierbaren Heißstellen, die neben technischen Fehlern auch eine Beeinträchtigung der Sicherrheit des Herstellungsverfahrens hervorrufen können.

Die Arbeitstemperatur eines Katalysators kann man durch den Zusatz von Promotoren und durch das Herstellungsverfahren beeinflussen. Als Promotoren haben sich zugesetzte Oxide, Hydroxide und Peroxide der Alkali- und Erdalkalimetalle als besonders vorteilhaft herausgestellt (US-A-2.404.438). In einer Reihe von Patentanmeldungen, beispielsweise DE-A-23 00 512, wird vor allem der Zusatz der schweren Alkalimetalle als Promotoren beschrieben. In weiteren Patentanmeldungen, beispielsweise DE-A- 19 20 976, wird unter den Erdalkalimetallen das Barium besonders als Promotor hervorgehoben.

In der DE-A- 27 33 688 wird ein Verfahren zur Herstellung eines silberhaltigen Trägerkatalysators beansprucht, wobei man ein Trägermaterial mit einer Silberverbindung imprägniert, die imprägnierten Teilchen durch zumindest teilweise Umwandlung in elementares Silber aktiviert und schließlich auf dem so hergestellten Katalysator mindestens eines der Alkalimetalle Kalium, Rubidium und Cäsium abscheidet. Die silberhaltige Imprägnierlösung enthält bevorzugt ein Bariumsalz.

Alle bisher beschriebenen Katalysatoren weisen die relativ hohe Arbeitstemperatur von 230 bis 260°C auf.

EP-A-428845 beschreibt ein Verfahren zur Herstellung eines Silberkatalysators, bei dem ein homogen poröser hitzebeständiger Träger mit einer silberhaltigen Lösung imprägniert wird und der imprägnierte Träger vorgetrocknet und thermisch formiert wird, gekennzeichnet durch
- Auswählen mäßig löslicher Silberverbindungen und -formulierungen, die bei 50 °C eine Lösung mit weniger als 400 g Silber pro Liter Lösung ergeben,
- Einstellen der Silberkonzentration der Imprägnierlösung auf einen Wert, bei dem die Konzentration der Silberverbindung mindestens 10 % unter der Sättigungskonzentration liegt,
- Einstellen der Temperatur beim Imprägnieren des Trägers auf einen Wert, bei der die Silberverbindung in Lösung bleibt,
- Vortrocknen des imprägnierten Trägers bei einer Temperatur von 40 - 120 °C,
- Thermisches Formieren des Trägers in einem Gasstrom, wobei während des Imprägnierens und Vortrocknens wiederholt evakuiert wird.

In EP-A-38 446 (= US-A-4.400.308) sind silberhaltige Trägerkatalysatoren beschrieben, bei deren Herstellung eine Ag-haltige Milchsäure auf den Träger getränkt wird. Die Milchsäure wird nach dem Trocknen in definierter Weise zweistufig in sauerstofffreier Atmosphäre vorzersetzt und bei der Aktivierung des Katalysators, ebenfalls in definierter Weise in sauerstoffhaltiger Atmosphäre endgültig zersetzt. Solche Katalysatoren erlauben Arbeitstemperaturen für die Ethylenoxid-Herstellung, die mit 160 bis 230°C deutlich tiefer sind als die des Standes der Technik; die Ethylenoxid-Selektivitäten betragen 80 bis 81 %.

Bei einer Weiterentwicklung der Katalysator-Herstellung von EP '446 wurde nun gefunden, daß ein Gehalt an Nitratanionen in der Tränkungslösung für den Katalysatorträger, die ansonsten weiterhin Milchsäure, Silberionen und gegebenenfalls Promotormetallionen enthält, Katalysatoren ergibt, die nochmals gesteigerte Ethylenoxid-Selektivitäten erreichen lassen. Dieser Befund ist angesichts EP '446 überraschend, da dort in der Vorzersetzung durch die Abwesenheit von Sauersoff eine nicht oxidierende Atmosphäre geschaffen wird und gemäß Beispiel 1 von EP '446 nitratfreies AgO eingesetzt wird.

Die Erfindung betrifft ein Verfahren zur Herstellung von silberhaltigen und gegebenenfalls Promotoren enthaltenden Trägerkatalysatoren durch Behandeln eines Trägers mit einer Silber und gegebenenfalls Promotormetalle enthaltenden Lösung, anschließende Trocknung und Kalzinierung, das dadurch gekennzeichnet ist, daß man
a) einen Träger mit einer spezifischen Oberfläche von höchstens 1,5 m²/g mit einer Lösung, die Milchsäure und Salpetersäure in freier Form oder in Form ihrer Anionen enthält, mit Silber- und gegebenenfalls Promotormetallionen in an sich bekannter Weise behandelt,
b) den nach a) erhaltenen behandelten Träger in einer praktisch sauerstofffreien Atmosphäre, die höchstens 100 Vol.-ppm O₂ enthält, bei 50 bis 120 °C trocknet und die in ionischer oder freier Form vorliegende Milchsäure und Salpetersäure im Temperaturbereich von 40 bis 220 °C und gegebenenfalls danach im Temperaturbereich von 400 bis 500 °C vorzersetzt, wobei man im Falle der zweistufigen Vorzersetzung für den Übergang zwischen den beiden Temperaturbereichen eine Aufheizrate von 70 bis 150°C/Stunde einstellt, und
c) das nach b) erhaltene, 0,5 bis 8 Gew.-% Kohlenstoff enthaltende Katalysator-Zwischenprodukt durch Erhitzen in sauerstoffhaltigen Atmosphäre aktiviert, wobei die Temperatur von mindestens 130 °C auf maximal 450 °C mit einer Aufheizrate von 3 bis 8 °C/Stunde gesteigert wird und der Sauerstoffgehalt von 0,4 bis 21 Vol.-% in einer solchen Weise gesteigert wird, daß im Abgas der Aktivierungsstufe ein CO₂-Gehalt von 2 Vol.-%, bevorzugt von 1 Vol.-% nicht überschritten wird.

Die erfindungsgemäß hergestellten Katalysatoren können solche mit oder ohne Promotoren sein. Für den Fall, daß die erfindungsgemäß hergestellten Katalysatoren Promotoren enthalten, seien hierfür Erdalkalimetallverbindungen, beispielsweise Verbindungen von calcium, Strontium oder Barium, und/oder Alkalimetallverbindungen, beispielsweise Verbindungen von Lithium, Natrium, Kalium, Rubidium oder Cäsium, genannt. Bevorzugte Promotoren sind Barium- und/oder Cäsium- und/oder Kalium-Verbindungen. Erfindungsgemäße Katalysatoren können Verbindungen des Bariums oder des Cäsiums oder Kaliums oder Verbindungen beider Metalle enthalten. Besonders bevorzugte erfindungsgemäß hergestellten Katalysatoren sind solche, die Barium- und Cäsium- und Kalium-Verbindungen enthalten.

Als Mengen für die aktiven Metalle seien beispielsweise die folgenden genannt, die alle als Metalle bzw. Metallionen gerechnet sind und sich alle auf das Gesamtgewicht des fertigen Katalysators beziehen: Für das Silber eine Menge von 7 bis 30 Gew.-%, bevorzugt 10 bis 20 Gew.-%, besonders bevorzugt 12 bis 18 Gew.-%; falls Erdalkalimetallverbindungen als Promotoren anwesend sind, hierfür eine Menge von 0,05 bis 0,5 Gew.-%, bevorzugt 0,07 bis 0,3 Gew.-%, besonders bevorzugt 0,08 bis 0,15 Gew.-%; für den Fall, daß Alkalimetalle als Promotoren anwesend sind, hierfür eine Menge von 0,001 bis 1 Gew.-%, bevorzugt 0,005 bis 0,5 Gew.-%, besonders bevorzugt 0,01 bis 0,05 Gew.-%; falls Erdalkalimetalle und Alkalimetalle gemeinsam als Promotoren anwesend sind, können sie unabhängig voneinander in den jeweils für sie angegebenen Mengen anwesend sein.

Als Trägermaterial für die erfindungsgemäß hergestellten Katalysatoren seien poröse, hitzebeständige Katalysatorträgermaterialien genannt, die sich unter den Bedingungen der Katalysator-Herstellung und der Katalysator-Verwendung inert verhalten. Das Trägermaterial hat eine makroporöse Struktur mit einer spezifischen Oberfläche von höchstens 1,5 m²/g, beispielsweise 0,01 bis 1,5 m²/g, bevorzugt 0,1 bis 1,1 m²/g, besonders bevorzugt 0,5 bis 1 m²/g. Die Porosität des Trägermaterials beträgt beispielsweise 40 bis 70 %, bevorzugt 45 bis 60 %. Für den Porendurchmesser sei beispielsweise der Bereich von 0,1 bis 100 µm genannt. Als Trägermaterialien mit den genannten physikalischen Eigenschaften kommen solche natürlichen oder synthetischen Ursprungs in Betracht, beispielsweise α-Aluminiumoxid, Siliciumcarbid, synthetische oder natürliche Zeolithe, Magnesiumoxid, Zirkoniumoxid oder keramische Materialien, bevorzugt α-Aluminiumoxid.

Die erfindungsgemäß hergestellten Katalysatoren sind dadurch gekennzeichnet, daß die auf den Träger aufzubringenden aktiven Metalle aus einer Lösung aufgebracht werden, die Milchsäure und Salpetersäure in freier Form oder in Form ihrer Anionen enthält. Die Menge an Anionen (Äquivalenten) der Milch- und Salpetersäure ist mindestens so groß wie die Summe an Äquivalenten der aktiven Metalle; dies stellt einen 100 %-Wert der Säureanionen dar. Der Gesamtbereich an Anionen beider Säuren (Milch- und Salpetersäure) beträgt 100 bis 200 %, bevorzugt 110 bis 150 % der Metalläquivalente in der soeben definierten Weise. Im Rahmen der Gesamtmenge beider Säuren ist deren Verhältnis zueinander so, daß pro 100 Mol-% (= Äquivalent-%) Milchsäure 1 bis 30 Mol-% (= Äquivalent-%), bevorzugt 3 bis 25 Mol-% Nitratanion vorliegen. Das Nitratanion kann in verschiedener Form der auf den Träger zu bringenden Lösung der aktiven Metalle zugesetzt werden. So kann einer gemäß EP '446 hergestellten Milchsäurelösung freie Salpetersäure (HNO₃) oder NH₄NO₃ zugesetzt werden. Weiterhin können Anteile der aktiven Metalle in Form ihres Nitrats zugesetzt werden, also ein Teil des Silberkations in Form von AgNO₃, Erdalkali- oder Alkalimetall in Form ihrer Nitrate, z.B. Ca(NO₃)₂, Sr(NO₃)₂, Ba(NO₃)₂, LiNO₃, NaNO₃, KNO₃, RbNO₃ oder CsNO₃ oder mehrere von ihnen. Ebenso kann aber auch der Träger vor dem Aufbringen der Lösung der aktiven Metalle mit HNO₃ oder NH₄NO₃-Lösung behandelt werden oder der Träger nach dem Aufbringen der Lösung der aktiven Metalle, gegebenenfalls nach einem Trocknungsvorgang, mit HNO₃ oder NH₄NO₃-Lösung behandelt werden, wobei in jedem Falle die Menge an Nitratanion der obigen Definition entspricht. Das jeweilige Aufbringen erfolgt in fachmännisch bekannter Weise, z.B. durch Tränken oder Aufsprühen.

Als Träger für das erfindungsgemäße Katalysator-Herstellungsverfahren sei beispielsweise einer der oben beschriebenen Träger mit den dort beschriebenen physikalischen Eigenschaften genannt. Bevorzugt wird α-Aluminiumoxid mit den oben beschriebenen Eigenschaften als Träger eingesetzt.

Als Milchsäure kann das Racemat, das die beiden optischen Antipoden in jeweils gleichen Mengen enthält, eingesetzt werden oder eine Milchsäure, die eines der optischen Antipoden im Überschuß enthält. Bevorzugt ist der Einsatz einer. Milchsäure, die neben einem Gehalt an Racemat eines der optischen Antipoden im Überschuß enthält. Für diese bevorzugte Form sei beispielsweise eine Milchsäure genannt, die mindestens 50 Gew.-% eines optischen Antipoden, bevorzugt mindestens 50 Gew.-% der L(+)-Form, besonders bevorzugt mindestens 80 Gew.-% der L(+)-Form enthält, wobei der Rest jeweils aus dem Racemat der beiden optisch aktiven Formen besteht. Milchsäure kann auch in Form des Lactats eines oder mehrerer der aktiven Metalle oder mindestens teilweise als Ammoniumlactat eingesetzt werden.

Die erfindungsgemäß zum Behandeln des Katalysatorträgers eingesetzte Milchsäure enthält Silber in Form von Silberionen. Für die Herstellung einer solchen silberhaltigen Milchsäure sei beispielsweise das Eintragen von Silberoxid, Silbercarbonat oder separat hergestelltem Silberlactat in die Milchsäure genannt. Selbstverständlich können auch andere in der Hitze zersetzliche Silberverbindungen hierfür verwendet werden. Im Rahmen der oben gegebenen Definition kann weiterhin ein Teil des Silbers in Form von AgNO₃ eingesetzt werden. Bevorzugt ist das Eintragen von Silberoxid, besonders bevorzugt von frisch gefälltem Silberoxid, in die Milchsäure. Die zum Behandeln verwendete Milchsäure enthält beispielsweise 25 bis 45 Gew.-%, bevorzugt 28 bis 40 Gew.-% Silberionen, bezogen auf die Gesamtmenge an Behandlungsflüssigkeit. Der gewünschte Gehalt an Silberionen kann gegebenenfalls vor dem Behandeln durch Zugabe von destilliertem Wasser, konzentrierter oder verdünnter HNO₃ oder verschieden hoch konzentrierter NH₄NO₃-Lösungen zur Behandlungsflüssigkeit auf das gewünschte Maß eingestellt werden. Die Herstellung der silberhaltigen Milchsäure erfolgt bei einer Temperatur von beispielsweise 40 bis 70°C. Nach Beendigung des Zusatzes der Silberverbindung wird der Milchsäure zweckmäßigerweise noch eine Menge von etwa 1 ml 30 gew.-%iger Wasserstoffperoxid-Lösung/100 g Silberionen zugesetzt.

Für den Fall, daß Erdalkali- und/oder Alkalimetall-Ionen als Promotoren zugesetzt werden, sei ein Zusatz von Hydroxiden, Carbonaten oder Nitraten eines oder mehrerer Erdalkali- und/oder Alkalimetalle genannt. Für die Erdalkalimetalle seien beispielsweise folgende Verbindungen genannt: Calciumhydroxid, Calciumcarbonat, Calciumnitrat, Strontiumhydroxid, Strontiumcarbonat, Strontiumnitrat, Bariumhydroxid, Bariumcarbonat, Bariumnitrat, bevorzugt Bariumhydroxid, Bariumcarbonat oder Bariumnitrat. Als Alkalimetallverbindungen seien beispielsweise genannt: Lithiumhydroxid, Lithiumcarbonat, Lithiumnitrat, Natriumhydroxid, Natriumcarbonat, Natriumnitrat, Kaliumhydroxid, Kaliumcarbonat, Kaliumnitrat, Rubidiumhydroxid, Rubidiumcarbonat, Rubidiumnitrat, Cäsiumhydroxid, Cäsiumcarbonat, Cäsiumnitrat, bevorzugt Cäsiumhydroxid, Cäsiumcarbonat oder Cäsiumnitrat. Als Menge sei beispielsweise bei den Erdalkalimetallverbindungen eine solche von 0,5 bis 3, bevorzugt 0,7 bis 2 g/100 g 100 %iger Milchsäure genannt. Für die Alkalimetallverbindungen sei beispielsweise eine Menge von 30 bis 300, bevorzugt 70 bis 200 mg/ 100 g 100 %iger Milchsäure genannt.

Die Menge der angesetzten, das Silber, das Nitratanion und gegebenenfalls die Promotormetalle enthaltenden Milchsäure sowie die Mengen der darin enthaltenen aktiven Metalle richten sich selbstverständlich nach der gewünschten Menge der aktiven Metalle auf dem fertigen Katalysator im Rahmen der obengenannten Bereiche für die einzelnen Metalle und weiterhin nach der Porosität des eingesetzten. Katalysatorträgers. Diese Zusammenhänge können jedoch in einfachen Vorversuchen ermittelt werden. Das Volumen der zum Behandeln eingesetzten Milchsäurelösung wird so bemessen, daß der Katalysatorträger vollständig besprüht oder getränkt wird und gegebenenfalls nach dem Tränken nur wenig Tränkungsflüssigkeit vom getränkten Katalysator abtropft. Beispielsweise sei als vom getränkten Träger abtropfende Milchsäurelösung eine Menge von 5 bis 30, bevorzugt 10 bis 20 Vol.-% der gesamten eingesetzten Milchsäurelösung genannt. Diese Menge kann in bekannter Weise vorab berücksichtigt werden.

In einer bevorzugten Variante des erfindungsgemäßen Verfahrens können der Silber, Nitratanionen und gegebenenfalls Promotormetalle enthaltenden Milchsäure lösliche, leicht zersetzliche, nicht reduzierende organische Verbindungen zugesetzt werden, wie Zuckeralkohole (Sorbit, Mannit), Polyhydroxysäuren, Saccharose, Stärke, Trimellithsäure, bevorzugt Saccharose. Diese genannten organischen Verbindungen werden beispielsweise in einer Menge von etwa 30 bis etwa 45 g/100 g 100 %iger Milchsäure zugesetzt.

Die Reihenfolge der Zugabe der einzelnen Komponenten für die Behandlungsflüssigkeit kann beispielsweise die folgende sein: Vorlegen der Milchsäure, gegebenenfalls Eintragen der Erdalkalimetallverbindung, gegebenenfalls Eintragen der Alkalimetallverbindung, Eintragen der Silberverbindung, Zugabe des Nitratanions in einer der genannten Formen, zweckmäßigerweise Zusatz des Wasserstoffperoxids und gegebenenfalls Zusatz der beschriebenen organischen Verbindung. Das Eintragen der genannten Komponenten in die vorgelegte Milchsäure kann jedoch auch in beliebiger anderer Reihenfolge erfolgen.

Mit der in der beschriebenen Weise erhaltenen Behandlungsflüssigkeit wird ein oben beschriebener Katalysatorträger durch ein- oder mehrmaliges Eintauchen in die Lösung oder Besprühen behandelt. Den beschriebenen Überschuß der Tränkungsflüssigkeit läßt man frei abtropfen und bringt sodann den behandelten Träger in einen Umluftofen.

In diesem Umluftofen wird eine praktisch sauerstofffreie Atmosphäre aufrecht erhalten. Als praktisch sauerstofffreie Atmosphäre sei eine solche genannt, die höchstens 100 Vol.-ppm O₂ enthält, beispielsweise 1 bis 100, bevorzugt 1 bis 20 Vol.-ppm. Als Inertgas für die praktisch sauerstofffreie Atmosphäre können beispielsweise Stickstoff, Kohlendioxid oder Edelgas, bevorzugt Stickstoff, verwendet werden, die sauerstofffrei sind oder deren Sauerstoffgehalt in dem genannten Bereich liegt.

In dem beschriebenen Umluftofen wird zur Trocknung des behandelten Trägers eine Temperatur von etwa 50 bis etwa 120°C, bevorzugt 90 bis 120°C, besonders bevorzugt 100 bis 110°C, eingestellt. Die Dauer des Trocknungsvorganges ist abhängig von der Menge des behandelten Trägers, der Menge Wasser in der Behandlungsflüssigkeit, der Menge des Umluftstromes und der Höhe der Temperatur innerhalb des angegebenen Bereiches. Diese Zeit kann im Bereich von etwa 1 bis etwa 4 Stunden liegen.

Danach wird der behandelte und getrocknete Katalysatorträger einer Vorzersetzung der organischen Bestandteile ebenfalls in praktisch sauerstofffreier Atmosphäre unterworfen. Hierzu wird die Temperatur in den Bereich von 140 bis 220, bevorzugt 140 bis 160°C, besonders bevorzugt 145 bis 155°C angehoben und für 1/2 bis 2 Stunden in diesem Bereich belassen. Gegebenenfalls wird danach mit einer Aufheizrate von 70 bis 150, bevorzugt 90 bis 110°C pro Stunde eine zweite Vorzersetzungstemperatur eingestellt. Diese zweite Temperatur liegt beispielsweise im Bereich von 400 bis 500, bevorzugt 430 bis 470°C und wird wie die erste Vorzersetzungstemperatur für 1/2 bis 2 Stunden eingehalten. Die Wirkungsweise des Nitratzusatzes und der beiden beschriebenen separaten Temperaturbereiche ist noch nicht abschließend untersucht. Es kann jedoch angenommen werden, daß im ersten der angegebenen Vorzersetzungstemperaturbereiche eine schonende Zersetzung des organischen Materials begonnen wird, wobei ein Zusammenwachsen von Silberkristallen zu größeren Einheiten weitgehend vermieden wird. Im zweiten der genannten Vorzersetzungstemperaturbereiche kann sodann eine weitere Zersetzung des organischen Materials und eine Entfernung der entstehenden flüchtigen Brenzprodukte angenommen werden. Für den Fall, daß Promotormetalle im Katalysator vorhanden sind, kann weiterhin angenommen werden, daß im zweiten der genannten Temperaturbereiche Festkörperreaktionen zwischen den Promotormetallverbindungen und dem Silber stattfinden.

Nach dieser erfindungsgemäßen Vorzersetzung enthält das dabei erhaltene Katalysator-Zwischenprodukt etwa noch 0,5 bis 8, bevorzugt 1 bis 8, besonders bevorzugt 2 bis 5, Gew.-% Kohlenstoff, bezogen auf das Gesamtgewicht des Katalysator-Zwischenprodukts.

Im Anschluß an die beschriebene Vorzersetzung wird das Katalysator-Zwischenprodukt durch Erhitzen in einer sauerstoff-haltigen Atmosphäre aktiviert..Für dieses Erhitzen wird das Katalysator-Zwischenprodukt zunächst in der praktisch sauerstofffreien Atmosphäre auf eine Temperatur von höchstens 130°C gebracht und sodann in Gegenwart von Sauerstoff bei einer von mindestens 130°C auf maximal 300°C ständig steigenden Temperatur behandelt, wobei eine Aufheizrate von 3 bis 8°C pro Stunde eingehalten werden muß. Bevorzugt wird der beschriebene Temperaturanstieg von mindestens 140 bis maximal 260°C, besonders bevorzugt von mindestens 150 bis maximal 240°C vorgenommen.

Zu Beginn des beschriebenen Aufheizprogrammes wird die praktisch sauerstofffreie Atmosphäre durch eine zunächst 0,4 Vol.-% Sauerstoff enthaltende Atmosphäre ersetzt, wobei der Rest zu 100 Vol.-% beispielsweise aus den obengenannten Inertgasen bestehen kann. Dieser Sauerstoffgehalt in der Aktivierungs-Atmosphäre wird sodann langsam von den genannten 0,4 bis auf 21 Vol.-% in einer solchen Weise gesteigert, daß im Abgas dieser Aktivierungsstufe ein CO₂-Gehalt von 2 Vol.-%, bevorzugt von 1 Vol.-% nicht überschritten wird. Die Dauer dieser erfindungsgemäßen Aktivierung kann aus der Aufheizgeschwindigkeit und dem gewählten Temperaturintervall abgeleitet werden und beträgt beispielsweise 12 bis 30 Stunden.

In einer Variante des beschriebenen Vorzersetzungs/Aktivierungsvorganges kann zunächst nur der erste Vorzersetzungsschritt im Temperaturbereich von 140 bis 200°C vorgenommen werden und unter Verzicht auf den zweiten Vorzersetzungschritt und nach Abkühlen auf 130°C oder darunter die weitere Vorzersetzung mit der beschriebenen Aktivierung in der für die Aktivierung beschriebenen Weise zusammengefaßt werden.

Die erfindungsgemäße Vorzersetzung und Aktivierung können unabhängig voneinander jeweils bei Normaldruck, Unter- oder Überdruck durchgeführt werden. Hierfür sei beispielsweise ein Bereich von 0,1 bis 50 bar, bevorzugt 1 bis 20 bar, besonders bevorzugt 5 bis 15 bar, genannt.

Im erfindungsgemäßen Verfahren ist es nicht erforderlich, die Vorzersetzung und die Aktivierung zum fertigen Katalysator unmittelbar hintereinander durchzuführen. Es ist also beispielsweise möglich, nach dem Durchlaufen des zweiten Vorzersetzungstemperaturbereiches in praktisch sauerstofffreier Atmosphäre das hierbei erhaltene Katalysator-Zwischenprodukt unter praktisch sauerstofffreier Atmosphäre bis auf 70 bis 80°C abzukühlen und sodann als Zwischenprodukt aus dem Umluftofen zu nehmen. Dieses Katalysator-Zwischenprodukt enthält wie beschrieben 0,5 bis 8, bevorzugt 1 bis 8, besonders bevorzugt 2 bis 5 Gew.-% Kohlenstoff, bezogen auf das Gesamtgewicht dieses Zwischenproduktes. Dieses Zwischenprodukt ist ohne Einbuße an katalytischer Aktivität des daraus herstellbaren aktiven Silberkatalysators beliebig lagerfähig. Das Zwischenprodukt wird vor seinem Einsatz zu dem endgültigen Katalysator aktiviert. Diese Aktivierung kann beispielsweise in dem beschriebenen Umluftofen unter den beschriebenen Bedingungen erfolgen.

Die Aktivierung des Katalysator-Zwischenproduktes kann jedoch auch in dem Reaktor erfolgen, in dem der nach der Aktivierung fertige Katalysator für die vorbestimmte katalytische Reaktion eingesetzt wird, beispielsweise in einem Reaktor zur Alkylenoxid-Herstellung, sofern ein solcher Reaktor die Einhaltung der oben beschriebenen Aktivierungsbedingungen in Bezug auf Temperaturführung und die Dosierung des Sauerstoffes ermöglicht. Diese zuletzt genannte Verfahrensweise zur Aktivierung des Katalysator-Zwischenprodukts ist bevorzugt.

Beispielsweise kann das Katalysator-Zwischenprodukt, gegebenenfalls nach einer längeren Zwischenlagerung, in das an sich bekannte Röhrensystem eines Festbettreaktors zur Ethylenoxid-Herstellung eingefüllt werden. Das Zwischenprodukt wird dann unter einem praktisch sauerstofffreien Inertgasstrom auf mindestens 130°C aufgewärmt. Das Aufheizen kann durch den Inertgasstrom erfolgen, jedoch auch durch ein um die Rohre zirkulierendes Wärmeträgermedium unterstützt werden. Sodannn wird die bereits beschriebene Steigerung der Temperatur mit der beschriebenen Aufheizrate vorgenommen und eine Sauerstoffkonzentration von anfänglich 0,4 Vol.-% eingestellt. Der Abgasstrom des Reaktors wird ständig auf einen CO₂-Gehalt überprüft, der erfindungsgemäß 2 Vol.-%, bevorzugt 1 Vol.-% nicht überschreiten soll. In der beschriebenen Weise wird sodann unter Einhaltung dieses CO₂-Gehaltes der Sauerstoffgehalt im Reaktoreingangsgas bis auf 21 Vol.-% gesteigert. Wenn nach Erreichen des oberen Temperaturwertes in der Aktivierungsstufe der CO₂-Gehalt des Reaktorabgases auf einen Wert von unter 0,3 Vol.-%, beispielsweise auf 0,1 abgesunken ist, wird die Aktivierung des Katalysators beendet. Die Temperatur im Reaktor wird sodann auf die für die Alkylenoxid-Herstellung benötigte Temperatur, beispielsweise die für die Ethylenoxid-Herzustellung benötigte Temperatur, abgesenkt und die Herstellung von Alkylenoxid, beispielsweise Ethylenoxid, durch Einleiten der dem Fachmann bekannten. Gasmischung zur Alkylenoxidherstellung in das Katalysatorbett begonnen.

Die Erfindung betrifft daher weiterhin ein Verfahren zur Herstellung der Zwischenprodukte für silberhaltige und gegebenenfalls Promotoren enthaltende Trägerkatalysatoren, welches durch die erfindungsgemäße und oben beschriebene Tränkung, Trocknung, Vorzersetzung und anschließende Abkühlung in praktisch sauerstofffreier Atmosphäre auf eine Temperatur von etwa 70 bis 80°C gekennzeichnet ist. Das Zwischenprodukt wird bevorzugt durch Anwendung der beschriebenen zweistufigen Vorzersetzung hergestellt.

Die erfindungsgemäß hergestellten Katalysatoren zeigen in Übereinstimmung mit EP '446 und im Vergleich zu den nach davor üblichen Herstellungsverfahren erhaltenen Katalysatoren deutliche Unterschiede in ihrer spezifischen Oberfläche, der Morphologie der Silberoberfläche und der Teilchengröße der Silberkristallite. So liegt die spezifische Oberfläche, gemessen nach den BET-Verfahren (J. Am. Chem. Soc. 60, 309-316 (1938)) bei etwa 0,2 bis 0,8 m²/g gegenüber einer spezifischen Oberfläche von weniger als 0,1 m²/g bei Katalysatoren, die nach üblichen Verfahren hergestellt wurden. Die mikroskopische Untersuchung zeigt bei den erfindungsgemäßen Katalysatoren eine gleichmäßige, kontinuierliche Teilchenstruktur mit sphärischen Silberkristalliten, die einen mittleren Durchmesser von 0,02 bis 0,4 µm aufweisen, während Katalysatoren, die nach den üblichen Verfahren erhalten worden sind, in der Morphologie der Silberoberfläche einen glasartigen Überzug mit Silberkristalliten zeigen, die einen mittleren Durchmesser von 0,7 bis 2 µm haben.

Die erfindungsgemäß hergestellten Katalysatoren sind beispielsweise für die Herstellung von Alkylenoxiden durch Dampfphasenoxidation von Olefinen mit Luft bzw. mit anderen molekularen Sauerstoff enthaltenden Gasen verwendbar. Als Alkylenoxide seien beispielsweise Ethylenoxid, Propylenoxid, 1,2-Butylenoxid oder 2,3-Butylenoxid, bevorzugt Ethylenoxid, genannt. Als hierzu einsetzbare Olefine seien beispielsweise Ethylen, Propylen, 1,2-Butylen oder 2,3-Butylen, bevorzugt Ethylen, genannt. Die Erfindung betrifft somit auch die Verwendung der erfindungsgemäß hergestellten Katalysatoren für die beschriebene Herstellung von Alkylenoxiden.

Diese erfindungsgemäße Verwendung ist durch die überraschend hohe Aktivität und hohe Selektivität in Bezug auf die Alkylenoxid-Ausbeute besonders vorteilhaft. So ist die Arbeitstemperatur bei der Ethylenoxid-Herstellung unter Verwendung der erfindungsgemäß hergestellten Katalysatoren bei 160 bis 230, bevorzugt 180 bis 220°C einstellbar, während übliche Arbeitstemperaturen bei Verfahren nach dem Stande der Technik bei 230 bis 260°C liegen. Die Selektivität beispielsweise für die Ethylenoxidherstellung liegt hierbei bei 83 bis 84 %.

Infolge der tieferen Arbeitstemperatur wird bei Verwendung der erfindungsgemäßen Katalysatoren die Bildung unerwünschter Nebenprodukte zurückgedrängt. Ebenso wird die Bildung von Heißstellen im Katalysatorbett zurückgedrängt, wobei gleichzeitig die Sicherheit des Alkylenoxid-Herstellungsprozesses gefördert wird.

Die erfindungsgemäße Vorzersetzung und Aktivierung des erfindungsgemäßen Katalysators verläuft unter äußerst schonenden Bedingungen, die zu den beschriebenen speziellen Eigenschaften der erfindungsgemäß hergestellten Katalysatoren führen. Im Gegensatz hierzu konnte bei den herkömmlichen Verfahren der unkontrollierten Zersetzung und Aktivierung von silberhaltigen Trägerkatalysatoren wegen der stark exotherm verlaufenden und durch das anwesende Silber in unkontrollierter Weise katalysierten Verbrennung von Kohlenstoff zu Kohlendioxid punktweise auftretende sehr hohe Temperaturen wegen der fehlenden Temperatur- und Sauerstoff-Kontrolle nicht vermieden werden. Solche Unregelmäßigkeiten bei der Katalysatorherstellung wirken sich jedoch sehr nachteilig auf die für die katalytische Aktivität verantwortliche Struktur der Silberkristalle aus. Der erfindungsgemäße Zusatz von Nitrationen bewirkt gegenüber EP '446 eine weiter gesteigerte kontrollierte Zersetzung, wodurch eine Agglomeration der Silberkristallite zugunsten kleinerer Silberpartikel weiter zurückgedrängt wird.

In der bevorzugten Verfahrensvariante, in der unter Benutzung des erfindungsgemäß hergestellten Katalysator-Zwischenproduktes die Aktivierung dieses Zwischenproduktes im Alkylenoxid-Reaktor vorgenommen wird, zeigt sich ein weiterer bedeutsamer Vorteil der vorliegenden Erfindung: der jedem Fachmann bekannte sehr kritische Umgang mit aktivierten Katalysatoren, beispielsweise beim Lagern, beim Transport oder beim Einfüllen in den Reaktor, entfallt hierbei. Gleichzeitig sind alle beim Umgang mit aktivierten Katalysatoren möglichen irreversiblen nachteiligen Beeinflussungen ausgeschlossen, so daß erfindungsgemäß hergestellte Katalysatoren nach der bevorzugten Verfahrensvariante in einem voll aktiven, durch nichts beeinträchtigten Zustand zum bestimmungsgemäßen Einsatz gelangen.

### Beispiel 1 Herstellung von Silber-Lactat

1600. g AgNO₃ DAB 7 wurden in einem 10 l Becherglas unter Rühren in 4000 ml H₂O gelöst. Aus 420 g Ätznatron p.A., gelöst in 4000 ml H₂O dest., wurde eine Natronlauge hergestellt. Das Silberhydroxid wurde innerhalb von ca. 2 Stunden durch langsames Zutropfen der Natronlauge zur AgNO₃-Lösung ausgefällt. Das ausgefällte Silberhydroxid wurde im gleichen Gefäß mit jeweils 4000 ml H₂O dest. nitratfrei gewaschen (ca. 15 Waschvorgänge). Zur Prüfung auf Nitrat wurde eine Probe Waschwasser entnommen und in einem Reagenzglas vorsichtig mit einer 0,5 %igen Diphenylaminlösung in konzentrierter Schwefelsäure unterschichtet. Zeigte sich an der Grenzschicht ein blauer Ring, mußte weiter ausgewaschen werden. Der Nitrattest konnte auch mittels Analysenstäbchen der Fa. Merck durchgeführt werden. Nachweisgrenze: kleiner als 10 ppm NO₃⁻. Das Silberhydroxid wurde sodann abgenutscht und kräftig abgepresst, damit nur wenig Feuchtigkeit im Filterkuchen zurückblieb.

In einem 5000 ml Becherglas wurden 1600 g 90 %ige Milchsäure (L+ Gehalt >85 %) vorgelegt. Die Milchsäure wurde auf ∼50°C vorgewärmt, und unter Rühren wurde portionsweise das frisch gefällte und feuchte Silberhydroxid aufgelöst. Die Temperatur sollte beim Lösevorgang 80°C nicht übersteigen. Erst nachdem das gesamte Silberhydroxid aufgelöst war, wurde bei 80 bis 85°C etwas Wasserstoffperoxid (35 %ig, ca. 2 bis 10 ml) zugesetzt, bis die Silberlactatlösung eine klare gelbe Farbe hatte. Ausbeute: ca. 3470 g Silberlactat mit ca. 30 % Silber.

Die warme Lösung wurde auf ein VA-Blech gegossen und abgedeckt; das Silberlactat wurde beim Abkühlen fest. Das Silberlactat wurde in einem dunklen Behälter aufbewahrt.

### Beispiel 2 Herstellung von Katalysator A (Vergleich)

In einem 600 ml Becherglas wurden 200 g Silberlactat aus Beispiel 1 mit 3 ml 35 %igem H₂O₂ versetzt und bei ca. 70°C aufgeschmolzen. Zu dieser Schmelze wurden 0,96 g Ba(OH)₂ x 8 H₂O, 95 mg Cs₂CO₃ und 150 mg K₂CO₃ gegeben. Mit der erhaltenen Silberlactatlösung wurden 200 ml eines käuflichen α-Al₂O₃-Trägers mit einer spezifischen Oberfläche von 0,82 m²/g (Träger A) getränkt. Nach Abtropfen überschüssiger Silberlactatlösung wurde der getränkte Träger in einem Umluftofen unter Stickstoff bei etwa 80°C 2 Stunden getrocknet. Der Restsauerstoffgehalt blieb hierbei unter 100 ppm.

Nach dem Trocknen wurde die Temperatur innerhalb von 2 Stunden auf 220°C angehoben und dann für 2 Stunden gehalten. Danach wurde mit einer Aüfheizrate von 100°C/h weiterhin unter Stickstoff bis auf 450°C aufgeheizt und diese Temperatur ebenfalls 1 Stunde gehalten und anschließend auf 70 bis 80°C abgekühlt. Dieses Zwischenprodukt, das noch etwa 3 Gew.-% Kohlenstoff enthielt, wurde in den Versuchsreaktor eingefüllt. Der Reaktor wurde mit Stickstoff beaufschlagt (Raum-Zeit-Geschwindigkeit: 200 bis 1000 l/h) und die Temperatur über das Heizmedium auf 150°C angehoben. Der Restsauerstoff im Stickstoff blieb hierbei unter 100 ppm.

Mit dem Inertgas wurde nun dosiert soviel Sauerstoff zugeführt, daß die Sauerstoffeingangskonzentration bei 0,4 bis 6 Vol.-% lag. Die Bildung von CO₂ wurde analytisch verfolgt. Der CO₂-Gehalt durfte 1 Vol.-% nicht überschreiten. Entsprechend dem CO₂-Gehalt wurde die Temperatur um ca. 5°C/h angehoben. Die Sauerstoff-Zudosierung wurde ebenfalls um ca. 1 Vol.-%/h erhöht. Nach Erreichung einer Endtemperatur von 240°C und eines Sauerstoffgehaltes von 21 Vol.-% wurde 4 bis 6 Stunden, nachdem der CO₂-Gehalt unter 0,1 Vol.-% gesunken war, die Aktivierung des Zwischenproduktes beendet. Die Temperatur im Reaktor wurde auf 160°C abgesenkt und das für die Umsetzung zu Ethylenoxid erforderliche Gasgemisch wurde über den Katalysator geleitet. Nach einer Konditionierungszeit von ca. 48 Stunden erreichte der so hergestellte Katalysator seine endgültige Aktivität und Selektivität. Der so hergestellte Katalysator erhielt die Bezeichnung A. Die Versuchsergebnisse stehen in Tabelle 1.

### Beispiel 3 Herstellung von Katalysator B

Es wurde ein Katalysator wie in Beispiel 2 hergestellt. Dem Silberlactat wurden neben den Salzen von Barium, Cäsium und Kalium noch 10 g Silbernitrat zugesetzt.

### Beispiel 4 Herstellung von Katalysator C (Vergleich)

Es wurde ein Katalysator wie in Beispiel 2 hergestellt. Es wurde ein anderer, ebenso käuflicher α-Al₂O₃-Träger mit einer spezifischen Oberfläche von 0,80 m²/g (Träger B) mit der Lösung getränkt.

### Beispiel 5 Herstellung von Katalysator D

Es wurde ein Katalysator hergestellt wie in Beispiel 4 mit dem Träger B. Dem Silberlactat wurden neben den Salzen von Barium, Cäsium und Kalium noch 10 g Silbernitrat zugesetzt.

### Beispiel 6 (Anwendungsbeispiel)

Der Laborversuchsreaktor bestand aus einem ölbeheizten Metallrohr mit einer lichten Weite von 20 mm und einer Länge von 500 mm. In diesen Reaktor wurden zunächst 20 ml Inertmaterial und anschließend 170 ml Katalysator eingefüllt. Die Laborversuche wurden bei atmosphärischem Druck durchgeführt. Die analytische Überwachung des Reaktorproduktgasstromes erfolgte kontinuierlich durch einen Prozeßgaschromatographen. Die Raum-Zeit-Geschwindigkeit betrug: 250 Raumteile Gas pro Raumteil Katalysator und Stunde. Das eingesetzte Gasgemisch für die Gasphasenoxidation am Katalysator bestand aus:

| | |
|---|---|
| C₂H₄ | 30 Vol.-% |
| O₂ | 8 Vol.-% |
| N₂ + Inerte | 62 Vol.-% |

Dem eingesetzten Gasgemisch wurde als Inhibitor 1 bis 2 ppm 1,2-Dichlorethan zugesetzt.

Im technischen Reaktor wird der N₂-Gehalt des Gasgemisches zum größten Teil durch Methan zersetzt. Das Gasgemisch besteht dort beispielsweise aus 30 Vol.-% C₂H₄, 8 Vol.-% O₂, 50 Vol.-% CH₄ und 12 Vol.-% N₂+Inerte.

**Tabelle 1**

| Katalysator | Temperatur (°C) | EOX (Vol.-%)* | Selektivität (%) |
|---|---|---|---|
| A | 195 | 2,22 | 82,86 |
| A | 205 | 2,20 | 83,11 |
| B | 195 | 2,26 | 83,19 |
| B | 205 | 2,20 | 83,50 |
| C | 190 | 2,26 | 82,80 |
| C | 205 | 2,20 | 83,30 |
| D | 190 | 2,26 | 83,10 |
| D | 205 | 2,20 | 83,70 |

| | | | |
|---|---|---|---|
| Die Katalysatoren zeigten nach 3-monatigem Betrieb bei den verschieden angegebenen Temperaturen keinen Aktivitätsverlust. * EOX = Ethylenoxidgehalt des den Reaktor verlassenden Gasstrom; zur Beurteilung der Katalysatoren wurde die Selektivität bei gleich eingestellten EOX-Gehalten herangezogen. | | | |

Katalysatoren mit dem erfindungsgemäßen Nitratgehalt in der Behandlungslösung steigern die Selektivität um 0,3 bis 0,4 Prozentpunkte, woraus sich ein beträchtlicher wirtschaftlicher Vorteil bezüglich der Menge an EOX ergibt.

## Patentansprüche

1. Verfahren zur Herstellung von silberhaltigen und gegebenenfalls Promotoren enthaltenden Trägerkatalysatoren durch Behandeln eines Trägers mit einer Silber und gegebenenfalls Promotormetalle enthaltenden Lösung, anschließende Trocknung und Kalzinierung, **dadurch gekennzeichnet, daß** man
a) einen Träger mit einer spezifischen Oberfläche von höchstens 1,5 m²/g mit einer Lösung, die Milchsäure und Salpetersäure in freier Form oder in Form ihrer Anionen enthält, mit Silber- und gegebenenfalls Promotormetallionen in an sich bekannter Weise behandelt,
b) den nach a) erhaltenen behandelten Träger in einer praktisch sauerstofffreien Atmosphäre, die höchstens 100 Vol.-ppm O₂ enthält, bei 50 bis 120 °C trocknet und die in ionischer oder freier Form vorliegende Milchsäure und Salpetersäure im Temperaturbereich von 40 bis 220 °C und gegebenenfalls danach im Temperaturbereich von 400 bis 500 °C vorzersetzt, wobei man im Falle der zweistufigen Vorzersetzung für den Übergang zwischen den beiden Temperaturbereichen eine Aufheizrate von 70 bis 150 °C/Stunde einstellt, und
c) das nach b) erhaltene, 0,5 bis 8 Gew.-% Kohlenstoff enthaltende Katalysator-Zwischenprodukt durch Erhitzen in sauerstoffhaltigen Atmosphäre aktiviert, wobei die Temperatur von mindestens 130 °C auf maximal 450 °C mit einer Aufheizrate von 3 bis 8 °C/Stunde gesteigert wird und der Sauerstoffgehalt von 0,4 bis 21 Vol.-% in einer solchen Weise gesteigert wird, daß im Abgas der Aktivierungsstufe ein CO₂-Gehalt von 2 Vol.-%, bevorzugt von 1 Vol.-% nicht überschritten wird.

2. Verfahren nach Anspruch 1, **dadurch gekennzeichnet, daß** die Salpetersäure bzw. das Nitratanion in 1 bis 30 Mol.-%, bevorzugt 3 bis 25 Mol.-% der molaren Menge an Milchsäure vorliegt.

3. Verfahren nach Anspruch 1, **dadurch gekennzeichnet, daß** eine Milchsäure mit einem Gehalt an optisch aktiver Milchsäure eingesetzt wird, daß bevorzugt in der eingesetzten Milchsäure mindestens 50 % aus der L(+)-Form besteht, während der Rest das Racemat darstellt.

4. Verfahren nach Ansprüchen 1-3 **dadurch gekennzeichnet, daß** die Aktivierung des Katalysatorszwischenproduktes, gegebenenfalls nach einer Zwischenlagerung des Zwischenprodukts, in einem Reaktor für die Alkylenoxid-Herstellung unter Druck, bevorzugt bei 10 bis 15 bar, vorgenommen wird.

5. Verfahren nach Ansprüchen 1-3, **dadurch gekennzeichnet, daß** der das Silberion, das Nitration und gegebenenfalls die Promotormetallionen enthaltenden Milchsäure vor. dem Tränken in Milchsäure lösliche, leicht zersetzliche und nicht reduzierende organische Verbindungen zugesetzt werden.

6. Verfahren nach Anspruch 1, **dadurch gekennzeichnet, daß** eine Barium und Cäsium und Kalium als Promotoren enthaltende Milchsäure eingesetzt wird.

7. Verfahren zur Herstellung eines silberhaltigen und gegebenenfalls Promotormetallverbindungen enthaltenden, 0,5 bis 8 Gew.-% Kohlenstoff enthaltenden Katalysator-Zwischenprodukts durch Behandeln eines Trägers mit einer Silber und gegebenenfalls Promotonnetalle enthaltenden Lösung und anschliessende thermische Behandlung, **dadurch gekennzeichnet, daß** man
a) einen Träger mit einer spezifischen Oberfläche von höchstens 1,5 m²/g mit einer Lösung, die Milchsäure und Salpetersäure in freier Form oder in Form ihrer Anionen enthält, mit Silber- und gegebenenfalls Promotormetallionen in an sich bekannter Weise behandelt,
b) den nach a) erhaltenen behandelten Träger in einer praktisch sauerstofffreien Atmosphäre, die höchstens 100 Vol.-ppm O₂ enthält, bei 50 bis 120 °C trocknet und die in freier Form sowie in Form ihrer Anionen vorliegenden Milchsäure und Salpetersäure im Temperaturbereich von 140 bis 220 °C und gegebenenfalls danach im Temperaturbereich von 400 bis 500 °C vorzersetzt, wobei man im Falle der zweistufigen Vorzersetzung für den Übergang zwischen beiden Temperaturbereichen eine Aufheizrate von 70 bis 150 °C/Stunde einstellt und anschliessende Abkühlung in praktisch sauerstofffreier Atmosphäre, die höchstens 100 Vol.-ppm O₂ enthält, auf etwa 70 bis 80 °C oder tiefer.

8. Verwendung der nach dem Verfahren nach Ansprüchen 1 - 6 hergestellten Katalysatoren zur Herstellung von Alkylenoxiden durch Oxidation von Alkenen mit Sauerstoff enthaltenden Gasen.

## Claims

1. A process for the preparation of supported catalysts containing silver and optionally promoters by treating a support with a solution containing silver and optionally promoter metals, followed by drying and calcining, **characterized by** the following steps:
a) treating a support having a specific surface area of at most 1.5 m²/g in a per se known manner with a solution which contains lactic acid and nitric acid in a free form or in the form of their anions and comprises silver and optionally promoter metal ions;
b) drying the treated support obtained in a) in a virtually oxygen-free atmosphere which contains at most 100 volume ppm of O₂ at from 50 to 120 °C, and predecomposing the lactic acid and nitric acid which is in an ionic or free form within a temperature range of from 40 to 220°C, optionally followed by predecomposition within a temperature range of from 400 to 500 °C, wherein a heating rate of 70 to 150°C/hour is set for the transition between the two temperature ranges in the case of the two-step predecomposition; and
c) activating the catalyst intermediate product obtained in b), which contains from 0.5 to 8% by weight of carbon, by heating in an oxygen-containing atmosphere, wherein the temperature is increased from at least 130 °C to a maximum of 450 °C at a heating rate of from 3 to 8°C/hour, and the oxygen content is increased from 0.4 to 21% by volume in such a way that a CO₂ content of 2% by volume, preferably 1% by volume, is not exceeded in the exhaust gas of the activation step.

2. The process according to claim 1, **characterized in that** said nitric acid or the nitrate anion is present at 1 to 30 mole percent, preferably 3 to 25 mole percent, of the moles of lactic acid.

3. The process according to claim 1, **characterized by** employing a lactic acid with such a content of optically active lactic acid that preferably at least 50% of the lactic acid employed consists of the L(+) form, the rest being the racemate.

4. The process according to claims 1 to 3, **characterized in that** the activation of the catalyst intermediate product, optionally following an intermediate storage of the intermediate product, is effected in a reactor for alkylene oxide preparation under pressure, preferably at 10 to 15 bar.

5. The process according to claims 1 to 3, **characterized in that** soluble, readily decomposable, non-reducing organic compounds are added to said lactic acid containing the silver ion, nitrate ion and optionally promoter metal ions before the soaking in lactic acid.

6. The process according to claim 1, **characterized in that** a lactic acid containing barium and cesium and potassium as promoters is employed.

7. A process for the preparation of a catalyst intermediate product containing silver and optionally promoter metal compounds and containing from 0.5 to 8% by weight of carbon by treating a support with a solution which contains silver and optionally promoter metals, followed by thermal treatment, **characterized by** the following steps:
a) treating a support having a specific surface area of at most 1.5 m²/g in a per se known manner with a solution which contains lactic acid and nitric acid in a free form or in the form of their anions and comprises silver and optionally promoter metal ions;
b) drying the treated support obtained in a) in a virtually oxygen-free atmosphere which contains at most 100 volume ppm of O₂ at from 50 to 120°C, and predecomposing the lactic acid and nitric acid which is in an ionic or free form within a temperature range of from 140 to 220 °C, optionally followed by predecomposition within a temperature range of from 400 to 500°C, wherein a heating rate of 70 to 150°C/hour is set for the transition between the two temperature ranges in the case of the two-step predecomposition, followed by cooling in a virtually oxygen-free atmosphere which contains at most 100 volume ppm of O₂ down to about 70 or 80°C or lower.

8. Use of the catalysts prepared by the process according to claims 1 to 6 for the preparation of alkylene oxides by oxidation with gases containing oxygen.

## Revendications

1. Procédé de préparation de catalyseurs supports contenant de l'argent et, le cas échéant, des promoteurs, par traitement d'un support avec une solution contenant de l'argent et, le cas échéant, des métaux promoteurs, puis par séchage et calcination,
caractérisé,
a) en ce que l'on traite, de façon en soi connue, un support ayant une surface spécifique maximum de 1,5 m²/g, avec une solution qui contient de l'acide lactique et de l'acide nitrique se présentant sous forme libre ou sous la forme de leurs anions, avec des ions d'argent et, le cas échéant, avec des ions de métaux promoteurs,
b) en ce que l'on sèche le support traité obtenu selon l'étape a), dans une atmosphère pratiquement sans oxygène qui contient au maximum 100 ppm en volume de O₂, à une température comprise entre 50°C et 120°C, et on prédécompose l'acide lactique et l'acide nitrique présents sous forme ionique ou sous forme libre, dans la plage de températures comprises entre 40°C et 220°C et, le cas échéant, ensuite, dans la plage de températures comprises entre 400°C et 500°C, où, dans le cas de la prédécomposition à deux étages, on règle une vitesse de chauffage comprise entre 70°C/heure et 150°C/heure, pour le passage entre les deux plages de températures, et
c) en ce que l'on active le précurseur de catalyseur obtenu selon l'étape b) et contenant entre 0,5 % en poids et 8 % en poids de carbone, par chauffage dans une atmosphère contenant de l'oxygène, où la température est augmentée, passant d'au moins 130°C à 450°C au maximum, à une vitesse de chauffage comprise entre 3°C/heure et 8°C/heure, et la teneur en oxygène est augmentée, passant de 0,4 % en volume à 21 % en volume, d'une manière telle que la limite supérieure d'une teneur en CO₂ de 2 % en volume, de préférence de 1 % en volume, ne soit pas dépassée dans les effluents gazeux de l'étape d'activation.

2. Procédé selon la revendication 1, **caractérisé en ce que** l'acide nitrique ou l'anion de nitrate est présent entre 1 % et 30 % en pourcentage molaire, de préférence entre 3 % et 25 % en pourcentage molaire de la quantité molaire d'acide lactique.

3. Procédé selon la revendication 1, **caractérisé en ce que** l'on utilise un acide lactique ayant une teneur en acide lactique optiquement actif, **en ce que** l'acide lactique utilisé est composé, de préférence, d'une partie égale à au moins 50 % se présentant la forme L(+), tandis que la partie restante est représentée par le racémique.

4. Procédé selon l'une quelconque des revendications 1 à 3, **caractérisé en ce que**, pour la production d'oxyde d'alkylène, l'activation du précurseur de catalyseur est effectuée dans un réacteur, sous pression, de préférence comprise entre 10 bar et 15 bar, cette activation étant effectuée, le cas échéant, après un stockage temporaire du précurseur.

5. Procédé selon l'une quelconque des revendications 1 à 3, **caractérisé en ce que**, avant l'imprégnation d'acide lactique, des composés organiques solubles, facilement décomposables et ne se réduisant pas, sont ajoutés à l'acide lactique contenant l'ion d'argent, l'ion de nitrate et, le cas échéant, les ions de métaux promoteurs.

6. Procédé selon la revendication 1, **caractérisé en ce que** l'on utilise un acide lactique contenant du baryum, du césium et du potassium servant de promoteurs.

7. Procédé de préparation d'un précurseur de catalyseur contenant de l'argent et, le cas échéant, des composés de métaux promoteurs et une quantité comprise entre 0,5 % en poids et 8 % en poids de carbone, par traitement d'un support avec une solution contenant de l'argent et, le cas échéant, des métaux promoteurs, puis par traitement thermique,
caractérisé
a) en ce que l'on traite, de façon en soi connue, un support ayant une surface spécifique maximum de 1,5 m²/g, avec une solution qui contient de l'acide lactique et de l'acide nitrique se présentant sous forme libre ou sous la forme de leurs anions, avec des ions d'argent et, le cas échéant, avec des ions de métaux promoteurs,
b) en ce que l'on sèche le support traité obtenu selon l'étape a), dans une atmosphère pratiquement sans oxygène qui contient au maximum 100 ppm en volume de O₂, à une température comprise entre 50°C et 120°C, et on prédécompose l'acide lactique et l'acide nitrique présents sous forme libre ainsi que sous la forme de leurs anions, dans la plage de températures comprises entre 140°C et 220°C et, le cas échéant, ensuite, dans la plage de températures comprises entre 400°C et 500°C, où, dans le cas de la prédécomposition à deux étages, on règle une vitesse de chauffage comprise entre 70°C/heure et 150°C/heure, pour le passage entre les deux plages de températures et, ensuite, dans une atmosphère pratiquement sans oxygène qui contient au maximum 100 ppm en volume de O₂, on effectue un refroidissement à une température comprise entre environ 70°C et 80°C ou à une température plus basse.

8. Utilisation des catalyseurs préparés d'après le procédé selon l'une quelconque des revendications 1 à 6, pour la production d'oxydes d'alkylène, par oxydation d'alcènes avec des gaz contenant de l'oxygène.
